Europäisches Patentamt

(19) European Patent Office

Office européen des brevets

(11) **EP 0 627 404 B1**

(12) **EUROPEAN PATENT SPECIFICATION**

(45) Date of publication and mention
of the grant of the patent:
**20.08.1997 Bulletin 1997/34**

(21) Application number: **94902781.7**

(22) Date of filing: **16.12.1993**

(51) Int. Cl.$^6$: **C07C 67/08**

(86) International application number:
**PCT/ES93/00100**

(87) International publication number:
**WO 94/13617 (23.06.1994 Gazette 1994/14)**

(54) **PROCESS FOR THE SELECTIVE PRODUCTION OF MONOESTERS OF DIOLS AND TRIOLS USING ZEOLITIC CATALYSTS**

VERFAHREN ZUR SELEKTIVEN HERSTELLUNG VON DIOL- ODER TRIOL-MONOESTERN UNTER VERWENDUNG VON ZEOLITHKATALYSATOREN

PROCEDE D'OBTENTION SELECTIVE DE MONOESTERS DE DIOLS ET TRIOLS UTILISANT DES CATALYSEURS ZEOLITIQUES

(84) Designated Contracting States:
**AT BE CH DE DK ES FR GB GR IE IT LI LU MC NL PT SE**

(30) Priority: **17.12.1992 ES 9202555**

(43) Date of publication of application:
**07.12.1994 Bulletin 1994/49**

(73) Proprietors:
• **CONSEJO SUPERIOR DE INVESTIGACIONES CIENTIFICAS**
  **E-28006 Madrid (ES)**
• **UNIVERSIDAD POLITECNICA DE VALENCIA**
  **46071 Valencia (ES)**
• **UNIVERSIDAD COMPLUTENSE**
  **E-28040 Madrid (ES)**

(72) Inventors:
• **ARACIL MIRA, José**
  **E-28020 Madrid (ES)**
• **CORMA CANOS, Avelino**
  **46022 VALENCIA (ES)**
• **MARTINEZ RODRIGUEZ, Mercedes**
  **E-28030 Madrid (ES)**
• **SANCHEZ MENENDEZ, Nieves**
  **E-28006 Madrid (ES)**

(74) Representative: **Ungria Lopez, Javier et al**
  **Avda. Ramon y Cajal, 78**
  **28043 Madrid (ES)**

(56) References cited:
  **EP-A- 0 198 243       EP-A- 0 406 767**

## Description

Alkylic esters of fatty acids of the oleic, palmitic, stearic type, etc. are used as special lubricants in the cosmetics industry and in the food industry. In the case of esters obtained by reaction with triols, such as for example glycerol, the tri- as well as the di-and the monoester have commercial usefulness. When esterifications are carried out catalyzing with mineral acids, a mixture of different esters that must be separated by distillation for the purpose of obtaining fractions enrichened in the mono or diester, depending on the use to be given to it, is obtained.

### Prior art

It has recently been seen (patent JP 87225866 (9/9/87)) that it is possible to prepare highly pure diglycerides with a high yield, using an immobilized lipase. Kitano et al. for Lion Corporation also described in EP 0 407 959 (16/1/91) with priority JP 17967489 (11/7/89) (11/7/89) a "Process to produce monoesters of a polyol and a fatty acid" by means of an enzymatic reaction that is carried out by using an immobilized thermostable lipase.

The problem has been attacked by transesterification of fatty esters with glycerol (DE 705203716950) using dry sodium carbonate. This reagent is described in patent ES 9001084 with priority JP 899844189 (18/4/89) of Montañola Martínez et al. for Kao Corporation "Process for the preparation of monoesters of fatty acids with glycol" wherein the esterification, at a low temperature, catalyzing is done with a mixture of carbonate and bicarbonate giving rise to a highly pure monoester capable of emulsifying crystals and useable as a cosmetic.

Use of an amine as a catalyst is described in patent EP O 178913 of priority US 660728 (15/10/84) of Godwin for Exxon Research and Engineering Company "Preparation of glycol monoesters."

Holderich et al. for BASF Akiengesellschaft in patent DE 3638010 (7/11/86) describe a "Process to obtain an ester of alkenecarboxylic acids" wherein the esterification is done between 50 and 450º C in the presence of zeolites /or phosphates as catalysts. The same authors and for the same firm describe in EP 0 312 921 of priority DE 3735755 (22/10/87) entitled "Process to obtain unsaturated monoesters" also using zeolites and/or phosphates as catalysts.

Heinrich et al. for Hoechst Akiengesellschaft describe in EP 0 406 767 of priority DE 3921917 (4/7/89) a "Process to produce some esters of $C_{22}$-$C_{40}$ monocarbonic acids" that permits obtainment of some esters with excellent thermal stability using an acid ion exchanger as a catalyst.

Ankner et al. for Neste Oxo Aktiebolag describe in EP 0 367 743 of priority SE 8803978 (2/11/88) a "Process to prepare branched 1,3 glycols and monoesters thereof" in which the catalytic system comprises a phase transfer catalyst and an alkaline substance.

Other patents about the obtainment of monoesters are the following:

EP-A-348 764 (DE-A-3821479, appl. date 25/6/88) discloses a method for preparing alkanetriol monoesters by reacting $\alpha,\omega$,-diols with alkenol esters in the presence of a radical-generating compound. The radical-generating compound can be, for example, oxygen and all peroxides and azo-compounds which are normally used by polymerization of olefinic monomers. Examples are azoisobutyronitrile, hydroperoxides such as tert-butylhydroperoxide, diacylperoxides such as dibenzoylperoxide, peroxy acids and corresponding esters, and dialkylperoxides.

EP-A-20 549 (corresponding to US Pat. Appln. 954,363 of 25/10/78 and to WO-A-80/00837) discloses how monoesters of symmetrical dicarboxylic acids and symmetrical diols can be prepared in high yields by treating a diacid or diol with a monohydric alcohol or monocarboxylic acid, respectively, in the presence of a substantial amount of water. The use of a strong acid catalyst ($Ka>10^{-2}$) is suggested, whereby sulfuric acid is given as an example of a suitable catalyst.

### Brief description of the invention

The present invention describes an esterification process of saturated and unsaturated fatty acids (preferably oleic, palmitic, stearic and isostearic acids), containing from 4 to 22 carbon atoms, with diols and triols (preferably glycerol), wherein large pore zeolites are used as catalysts, and preferably with twelve member rings, and with acid or base features, obtaining the corresponding monoesters with high yields and selectivities.

The zeolites can be used directly as a catalyst or included in a matrix, of the different types cited in the bibliography among which, as examples, matrices of $Al_2O_3$, silica, kaolin and amorphous silica-alumina, stand out.

### Detailed description of the invention

The present invention refers to the use of zeolites, of specific characteristics, to direct the esterification between a monocarboxylic acid and a diol or triol to obtain a proportion higher to 88% of the monoester.

The esterification reaction takes place according to conventional processes in a continuous or discontinous reactor, of the agitated tank or fixed bed type, in which the zeolitic catalyst is found. The reaction is carried out at the temperature range of 60º C to 250º C, preferably between 100 and 190º C.

It is convenient to carry out the reaction under pressure (at 200 kPa or more) when low molecular weight alcohols are used or under a vacuum (5 kPa or less) when the water formed is to be eliminated by evaporation.

In the event that the balance is shifted by elimination of one of the reaction products, for example water, conversions higher than 80 % are achieved, the time required to reach this conversion being from 10 to 900 minutes, though this time is normally between 60 and 600 minutes.

The catalysts that the present invention refers to are zeolites, specifically of large pores with 12 member rings, since the geometric restrictions, due to the size of the pores, make it possible to obtain monoesters with high selectivities.

Catalysts based on zeolites that comply with the above specifications are, among others, the following (the abbreviations by parenthesis are the ones accepted by the International Zeolite Association (see W.M. Meier and D.H. Olson, Atlas of Zeolite Structure Types, 1992)):

- Faujasite (FAU),
- Mordenite (MOR),
- Commercial zeolites named ZSM-12, (MTW), omega (MAZ) and beta (BEA)
  which have shown good activity and selectivity.

Likewise, ALPOS and SAPOS ALPO-5 (AFI) and SAPO-5, SAPO-37 and SAPO-40 zeolites are also applicable.

In the esterification reaction, these catalysts act, in their acid as well as base form and as molecular molecular seives due to the pore size as it has already been explained. The zeolite, prior to its use, has to be prepared in one of these two forms, acidic or basic.

Preparation of the acidic form.

In the acidic form it is prepared either directly by exchange with a mineral acid, when the stability of the zeolite allows this, or else by an indirect method consisting of exchanging the alkaline and alkaline earth cations thereof by $NH_4^+$ by means of treatment thereof with an aqueous ammonium chloride solution, calcining the product obtained (using the so-called "deep bed" technique) or subjecting it to the action of steam if it is also desired to dealuminize the framework, operation that can also be done by chemical treatments with $(NH_4)_2F_6Si$, $SiCl_4$ or EDTA.

Acidic faujasites, prepared in this way, have an optimal activity for Si/Al ratios in the framework in the range of 4-30.

The activity of the catalyst also depends on the size of the crystals that the molecular seives form; the activity increases as the size of the crystal reduces. Hence, in the case of faujasites, an acid zeolite (HY), when its crystal size is 0.30 $\mu$m, is more active than when its crystal size is 0.80 $\mu$m; a beta zeolite, with a Si/Al ratio = 13, with a crystal size of 0.17 $\mu$m, is more active than one that has a crystal size of 0.80 $\mu$m.

In thse cases in which the zeolite contain $Na^+$ and/or $K^+$ as the exchange cation, the content thereof in the final acid catalyst, given as $Na_2O$, or $K_2O$, must be less than 5% by weight and preferably below 1% by weight, to avoid the resulting acidity from being very low.

Preparation of the basic form

The zeolitic catalyst, in its base form, is prepared by introducing alkaline cations into octahedral positions, by means of ionic exchange in liquid or solid phase.

The catalysts obtained from zeolites and SAPOS using this ion exchange technique with alkaline ions have the following order of activity according to the cation

$$Cs > K > Na > Li$$

It should be pointed out that good results are obtained with exchanged samples, in which there is a slight excess over the alkaline metal exchange capacity.

Examples

Example 1. Activity of an acid zeolite HY, with a unit cell of 24.43 Å and a content of $Na_2O$ of 0.10 %

The reaction is carried out in a reactor of a complete mixture of 100 ml. capacity, provided with a system to distill under a vacuum. The oleic acid and glycerol in a molar ratio 1:1 and the zeolitic catalyst 0.3% by weight are introduced in it.

The reaction temperature was kept at 180º C, the working pressure at 55 mmHg (7kPa) and the stirring speed of 600 r.p.m.

After 4 hours of reaction, the conversion into acid was 85%, with a selectivity in the monoester formation of 90%, the rest formed by diester, and less than 1% triester.

Example 2. Influence of the nature of the zeolite

The reaction is carried out using a beta zeolite as a catalyst, with a Si/Al ratio = 13, and $Na_2O + K_2O$ content of 0.01% by weight, in the same conditions as in example 1 and with the same proportions of reagents and catalyst.

After four hours of reaction, the conversion into acid was 80% with a monoester selectivity of 87%.

Example 3. Use of a zeolite with 12 member rings (acid mordenite)

Mordenite, unlike the zeolites used in examples 1 and 2, is unidirectional, and with a small pore diameter. The Si/Al ratio of the mordenite was 9. The acid and dealuminized form was obtained by treatment with hydrochloric acid of a synthesis mordenite with a Si/Al ratio = 5.

The reaction was carried out under the same conditions and with the same proportions of reagents as in the above two examples. The conversion after four hours was 66%, with a monoester selectivity of 82%.

Example 4. Influence of the reaction temperature

The same as in example 3 was used as a catalyst, the proportions of reagents and catalyst being the same as in said example.

The reaction conditions were the same as in example 1, except the reaction temperature that was increased from 180º C to 185º C. In these conditions, after four hours, the conversion was of 84% with a monoester selectivity of 83%.

Example 5. Effect of the framework composition of the zeolite on activity and selectivity.

The reaction was carried out under the same conditions as those of example 3 but using a mordenite with a Si/Al ratio = 12 as a catalyst. In this case, after five hours, the conversion was of 92% with a monoester selectivity of 87%.

Example 6. Capacity of base sodium zeolites to selectively produce the monoester.

The reaction was carried out under the same conditions and in the same proportion of reagents and catalyst as that of example 1, but using a NaY zeolite with a Si/Al ratio = 2.4, which, also, underwent an exchange process in a 0.1 N NaCl solution, with a solid/liquid ratio = 0.2 at room temperature for 30 minutes.

The conversion after five hours was 91%, with a monoester selectivity of 88%.

Example 7. Influence of the cation on the activity of the base zeolite.

A catalyst was prepared with base properties, exchanging a NaY zeolite with a Si/Al ratio = 2.4, in an O.1 M CsCl. with a S/L ratio = 0.2, at room temperature, for one hour.

The NaCsY zeolite thus prepared was used in the same conditions and proportions as those of example 6. The conversion obtained after five hours was 94%, with a monoester selectivity of 88%.

**Claims**

1. Process for the selective production of monoesters of diols and trials using zeolitic catalysts by means of selective esterification of a fatty acid and a polyalcohol, the fatty acid being selected from saturated and unsaturated fatty acids, containing from 4 to 22 carbon atoms, the polyalcohol being a dial or a triol, characterized in the use of large pare zeolites with acid or base characteristics, the reaction being carried out at a temperature between 60 and 250 °C.

2. A process according to claim 1, further characterized in that the reaction is carried out at a temperature between 100 and 190 °C.

3. Process according to claim 1 or 2, further characterized in that the reaction is carried out at pressures between 5kPa and 200 kPa.

4. Process according to claim 1, further characterized in that the zeolite used as the catalyst is prepared in acid or base forms thereof.

5. Process according to claim 1 or 4, further characterized in that the zeolite used as a catalyst is a faujasite.

6. Process according to claim 1 or 4, further characterized in that the zeolite used as a catalyst is a mordenite.

7. Process according to claim 1 or 4, further characterized in that the zeolite used as a catalyst is one of the following commercial names: ZSM-12; MCM-22, omega zeolite or beta zeolite.

8. Process according to claim 1 or 4, further characterized in that the zeolite used as a catalyst is one of the following commercial names: ALPOS or SAPOS in any of the forms SAPO-5, SAPO-37 or SAPO-40 thereof.

9. Process according to any of claims 1-8, further characterized in that the zeolites are used as a catalyst directly or included in a matrix.

10. Process according to any of the preceding claims, further characterized in that the large pore zeolites are large pore zeolites with twelve member rings.

11. Process according to any of the preceding claims, further characterized in that the fatty acid is oleic acid.

12. Process according to any of claims 1-10, further characterized in that the fatty acid is palmitic acid.

13. Process according to any of claims 1-10, further characterized in that the fatty acid is stearic acid.

14. Process according to any of claims 1-10, further characterized in that the fatty acid is isostearic acid.

15. Process according to any of the preceding claims, further characterized in that the polyalcohol is a diol.

16. Process according to any of claims 1-14, further characterized in that the polyalcohol is a triol.

17. Process according to claim 16, further characterized in that the triol is glycerol.

## Patentansprüche

1. Verfahren zur selektiven Herstellung von Monoestern von Diolen und Triolen unter Verwendung zeolithischer Katalysatoren durch selektive Veresterung einer Fettsäure und eines Polyalkohols, wobei die Fettsäure aus gesättigten und ungesättigten Fettsäuren, die 4 bis 22 Kohlenstoffatome enthalten, ausgewählt ist und der Polyalkohol ein Diol oder ein Triol ist, **gekennzeichnet durch** die Verwendung von großporigen Zeolithen mit sauren oder basischen Eigenschaften, wobei die Reaktion bei einer Temperatur zwischen 60 und 250 °C durchgeführt wird.

2. Verfahren nach Anspruch 1, weiterhin **dadurch gekennzeichnet**, daß die Reaktion bei einer Temperatur zwischen 100 und 190 °C durchgeführt wird.

3. Verfahren nach Anspruch 1 oder 2, weiterhin **dadurch gekennzeichnet**, daß die Reaktion bei Drücken zwischen 5 kPa und 200 kPa durchgeführt wird.

4. Verfahren nach Anspruch 1, weiterhin **dadurch gekennzeichnet**, daß der als Katalysator verwendete Zeolith in saurer oder basischer Form davon hergestellt wird.

5. Verfahren nach Anspruch 1 oder 4, weiterhin **dadurch gekennzeichnet**, daß der als Katalysator verwendete Zeolith ein Faujasit ist.

6. Verfahren nach Anspruch 1 oder 4, weiterhin **dadurch gekennzeichnet**, daß der als Katalysator verwendete Zeolith ein Mordenit ist.

7. Verfahren nach Anspruch 1 oder 4, weiterhin **dadurch gekennzeichnet**, daß der als Katalysator verwendete Zeolith ein Zeolith mit einem der folgenden Handelsnamen: ZSM-12; MCM-22, Omega-Zeolith oder Beta-Zeolith ist.

8. Verfahren nach Anspruch 1 oder 4, weiterhin **dadurch gekennzeichnet**, daß der als Katalysator verwendete Zeolith ein Zeolith mit einem der folgenden Handelsnamen: ALPOS oder SAPOS in einer der Formen SAPO-5, SAPO-37 oder SAPO-40 davon ist.

9. Verfahren nach einem der Ansprüche 1 bis 8, weiterhin **dadurch gekennzeichnet**, daß die Zeolithe als Katalysator direkt oder in einer Matrix eingeschlossen verwendet werden.

10. Verfahren nach einem der vorhergehenden Ansprüche, weiterhin **dadurch gekennzeichnet**, daß die großporigen Zeolithe großporige Zeolithe mit Zwölfer-Ringen sind.

11. Verfahren nach einem der vorhergehenden Ansprüche, weiterhin **dadurch gekennzeichnet**, daß die Fettsäure Ölsäure ist.

12. Verfahren nach einem der Ansprüche 1 bis 10, weiterhin **dadurch gekennzeichnet**, daß die Fettsäure Palmitinsäure ist.

13. Verfahren nach einem der Ansprüche 1 bis 10, weiterhin **dadurch gekennzeichnet**, daß die Fettsäure Stearinsäure ist.

14. Verfahren nach einem der Ansprüche 1 bis 10, weiterhin **dadurch gekennzeichnet**, daß die Fettsäure Isostearinsäure ist.

15. Verfahren nach einem der vorhergehenden Ansprüche, weiterhin **dadurch gekennzeichnet**, daß der Polyalkohol ein Diol ist.

16. Verfahren nach einem der Ansprüche 1 bis 14, weiterhin **dadurch gekennzeichnet**, daß der Polyalkohol ein Triol ist.

17. Verfahren nach Anspruch 16, weiterhin **dadurch gekennzeichnet**, daß das Triol Glycerin ist.

## Revendications

1. Procédé pour la production sélective de monoesters de diols et de triols à l'aide de catalyseurs zéolitiques par estérification sélective d'un acide gras et d'un polyalcool, l'acide gras étant choisi parmi les acides gras saturés et insaturés contenant de 4 à 22 atomes de carbone, le polyalcool étant un diol ou un triol, caractérisé par l'utilisation de zéolites à grands pores à caractéristiques acides ou basiques, la réaction étant conduite à une température comprise entre 60 et 250°C.

2. Procédé selon la revendication 1, caractérisé en outre en ce que la réaction est conduite à une température comprise entre 100 et 190°C.

3. Procédé selon la revendication 1 ou 2, caractérisé en outre en ce que la réaction est conduite à des pressions comprises entre 5 kPa et 200 kPa.

4. Procédé selon la revendication 1, caractérisé en outre en ce que la zéolite utilisée comme catalyseur est préparée sous ses formes acides ou basiques.

5. Procédé selon la revendication 1 ou 4, caractérisé en outre en ce que la zéolite utilisée comme catalyseur est une faujasite.

6. Procédé selon la revendication 1 ou 4, caractérisé en outre en ce que la zéolite utilisée comme catalyseur est une mordénite.

7. Procédé selon la revendication 1 ou 4, caractérisé en outre en ce que la zéolite utilisée comme catalyseur porte l'un des noms commerciaux suivants : ZSM-12, MCM-22, zéolite oméga ou zéolite béta.

8. Procédé selon la revendication 1 ou 4, caractérisé en outre en ce que la zéolite utilisée comme catalyseur porte l'un des noms commerciaux suivants : ALPOS ou SAPOS sous l'une quelconque de ses formes SAPO-5, SAPO-37 ou SAPO-40.

9. Procédé selon l'une quelconque des revendications 1 à 8, caractérisé en outre en ce que les zéolites sont utilisées comme catalyseur directement ou incluses dans une matrice.

10. Procédé selon l'une quelconque des revendications précédentes, caractérisé en outre en ce que les zéolites à grands pores sont des zéolites à grands pores à cycles à 12 chaînons.

11. Procédé selon l'une quelconque des revendications précédentes, caractérisé en outre en ce que l'acide gras est l'acide oléique.

12. Procédé selon l'une quelconque des revendications 1 à 10, caractérisé en outre en ce que l'acide gras est l'acide palmitique.

13. Procédé selon l'une quelconque des revendications 1 à 10, caractérisé en outre en ce que l'acide gras est l'acide stéarique.

14. Procédé selon l'une quelconque des revendications 1 à 10, caractérisé en outre en ce que l'acide gras est l'acide isostéarique.

15. Procédé selon l'une quelconque des revendications précédentes, caractérisé en outre en ce que le polyalcool est un diol.

16. Procédé selon l'une quelconque des revendications 1 à 14, caractérisé en outre en ce que le polyalcool est un triol.

17. Procédé selon la revendication 16, caractérisé en outre en ce que le triol est le glycérol.